# EUROPEAN PATENT SPECIFICATION

(11) **EP 0 598 219 B1**
(45) Date of publication and mention of the grant of the patent: **17.12.1997**
(21) Application number: 93116614.4
(22) Date of filing: 14.10.1993
(51) Int. Cl.: A61B 17/04, A61B 17/12

(54) **Suture securing device**
Vorrichtung zur Befestigung von Nahtmaterial
Dispositif de blocage pour l'immobilisation d'un fil de suture

(30) Priority: 17.11.1992 AU PL5843/92
(43) Date of publication of application: 25.05.1994
(73) Proprietor: SMITH & NEPHEW, INC., Memphis, Tennessee 38116 (US)
(72) Inventor: Martin, Christopher, Guildford, NSW (AU)
(74) Representative: Wächtershäuser, Günter, Prof. Dr.

(56) References cited:
- EP-A- 0 492 172
- FR-A- 2 178 908
- US-A- 3 880 166

## Description

The present invention relates generally to surgical devices, more particularly it relates to a suture securing device, an apparatus for delivery of such a device. It will be convenient to hereinafter refer to the suture securing device in relation to endoscopic surgery however it should be noted that the invention has a wider application.

The basic principles of surgery are incision, dissection, traction, counter-traction, ligation and suturing. These principles are well established and mastered in conventional "open" invasive surgical procedures where access to the surgical site is gained by incision through the patient's skin and body wall to expose the site. In recent times attention has been directed towards least invasive surgical procedures or non-invasive procedures such as endoscopy, which encompasses arthroscopy, laparoscopy, duodenoscopy, gastroscopy and the like, where access to the surgical site is gained by introducing surgical instruments down one or more portals and/or gastrointestinal tract from the mouth or anus and/or urinary tract via the urethra. Least invasive surgery, where possible, has many benefits over conventional invasive procedures such as reduced surgical trauma, lessened anaesthesia and shortened recovery times, leading to reduced hospital costs, greater patient comfort and earlier return to work.

Although some of the technology for laparoscopic surgery has been available for many years, the first laparoscopic cholecystectomy performed in 1987 has stimulated the interest of general surgeons in laparoscopic surgery. Many general surgeons have transferred the basic principles of surgery to laparoscopic and endoscopic surgery and have mastered with reasonable ease these principles with the exception of suturing. In endoscopic procedures difficulty arises in tying of a suture as the surgeon can only use instruments to manipulate the required knots. Manipulation of the suture is hindered as usually the surgeon has only a two dimensional viewing field - the suture site being visible by use of a camera and television screen or specialized scope. The problem of suturing and especially tying knots during laparoscopic surgery has become more apparent as surgeons have moved from resectional surgery i.e. where part of an organ or tissue is completely or partially removed such as cholecystectomy or appendectomy, to surgery which requires suture placement such as procedures of fundoplication, bowel resection etc. Resectional procedures such as cholecystectomy and appendectomy generally only require ligature or clip application to tie off the removed organ or blood vessel. Devices have been developed to facilitate intracorporeal placement of such clips or ligatures and the application of such ligatures or clips is relatively simple when compared to tying of intracorporeal knots in sutures. At the present time the average surgeon is not confronted with the need for intracorporeal knot tying frequently enough to become adept at this skill and this may contribute to longer operating times. Considerable time and practice may be required before a surgeon becomes skilled at tying of knots during endoscopic surgery.

A method for tying knots extracorporeally has been developed where the slip knot is tied in the suture outside the body of the patient and then positioned at the desired site by means of a push-rod into the body. While this method is successful to some extent, it suffers from a number of disadvantages. Many endoscopic procedures involve the inflation of a body cavity with a pressurized gas such as carbon dioxide so that a space is created in the body to make the surgical site visible. The gas pressure is kept constant to maintain the visual field and if the pressure is reduced by the escape of gas the visual field is lost or impaired as the body tissues resume their usual positions. In tying a knot in a suture outside the body it is necessary to pass the knot beyond a seal established to create the internal gas space. This involves breaking of the gas seal and the temporary loss of the gas space and hence the visual field as gas escapes when the suture is being passed into the body.

Secondly, the extracorporeally tied knots are not always as secure as would be desirable and thus present the risk of failure.

Thirdly, extracorporeally tied knots are not always satisfactory for securing opposite ends of a suture which closes an elongate wound.

US-A-3880166 discloses a device for occlusion of small blood vessels which includes a pad having one end thereof fastened to a length of surgical tape. The other end of the pad is designed to releasably maintain one end of the tape such that this end of the tape and the pad can occlude a blood vessel.

Accordingly, it is an object of the present invention to overcome, or at least alleviate, one or more of the difficulties and deficiencies related to the prior art.

Other objects and advantages of the invention will become apparent from the following description. This object is achieved by the device of claim 1.

The passage is configured such that a suture, looped through the passage of the securing device and having a free end passed about the periphery of the device and through the loop, can be drawn from the tissue abutting side to the suture loop side but not from the suture loop side to the tissue abutting side. Generally the passage will accommodate two sections of suture side by side but will not accommodate four sections of suture. Therefore if a loop of suture which emerges from the passage on the suture loop side has a length of suture passed through the loop, the loop will clamp upon that length of suture if the loop is retracted back into the passage.

At one or both ends of the passage the openings may be enlarged to positively seat the loop therein. Preferably there is a tapered recess for example a counter-bore at least about the opening in the suture loop side.

In a further preferred form the suture securing device further includes a suture accommodating channel in its outer peripheral surface. The channel is configured such that when said body is gripped about a portion of its outer peripheral surface, the portion of suture passing about the outer surface between the passage openings, and located in the channel is not also gripped. The channel may be adapted to receive all or part of the suture which passes about the periphery of the device when the suture is appropriately positioned. Preferably the channel is provided such that when the suture securing device has a suture positioned about it, the device can be manipulated by a clamp which grips the periphery of the body but the suture within the channel is still able to slide freely underneath the clamp so that the suture receiving device can be positioned accordingly. Preferably the channel is parallel to the passage.

In a still further preferred form, the suture securing device may further include a second suture passage extending through the surgically implantable body. The second suture passage may be positioned along the axis of the device from the first suture passage and preferably substantially parallel to the first passage.

In one embodiment the diameter of the second bore is only marginally larger than the diameter of the selected suture so that a knot in a suture will not pass through the passage.

In an alternative embodiment the second suture passage may be of the same dimensions and/or have the same functional features as the first suture passage. In this embodiment the suture securing device may be utilized to secure two separate sutures or alternatively both ends of one suture so as to form a ligature. The two passages may be of different dimensions such that two different types of suture may be secured. In this embodiment where two passages are provided the channel may be configured so that it accommodates portions of both sutures secured.

The suture receiving device may be adapted for releasable engagement with an apparatus for delivering the device in use to a surgical site. Accordingly the suture receiving device may include at least one locating means to cooperatively engage with an apparatus for positioning the device. A suitable delivery apparatus for positioning the device is described below.

The locating means may include a surface adapted to be contacted by the apparatus to push the device to bring the tissue abutting side into abutment with the tissue.

The locating means may include a pair of locating recesses at opposite ends of the surgically implantable body, each recess receiving a projecting portion of the apparatus upon engagement therewith. The locating recesses may be such that the device is held securely by the apparatus until intentionally released therefrom.

The body of the device may be elongate and may have a central axis. The suture passage may be a transverse passage which bisects the central axis.

A method for securing a suture includes
providing a suture securing device including
   a surgically implantable body having a tissue abutting side and a suture loop side; and
   a first suture passage through said body, said passage extending from an opening in the tissue abutting side to an opening in the suture loop side; and
a length of suture having a free end;
passing a loop of suture to be secured through said passage from the tissue abutting side to the suture loop side;
passing the free end of said suture around the device and through the loop;
drawing taut the free end of portion of suture passed through said loop; and
transferring the device along the suture to a desired position.

In a further embodiment of the present invention there is provided a suture apparatus including
a suture securing device as described above; and
a suture recovery means adapted to draw a loop of suture through said first suture passage.

Preferably the suture recovery means includes a snare shaped to pass through the passage in the suture securing device and retractable through said passage such that when said snare is passed through said passage and a section of suture is snared, the snare may be retracted through said passage bringing with it a loop of said suture.

More preferably the snare is attached to a disposable body.

In a still further embodiment of the present invention there is provided a suture apparatus including
a suture securing device according to claim 1; and
a length of suture having a free end and including a loop of suture positioned within the passage and extending beyond the suture loop side; the free end passing through said loop.

Preferably the free end of the suture has a suturing needle connected thereto.

In a further aspect there is provided in combination with the device of the present invention an apparatus for delivering a suture securing device as described above to a desired surgical site, including
an elongate hollow body adapted at one end to releasably engage said suture securing device;
a suture recovery means within said elongate hollow body adapted to draw a loop of suture through the first suture passage of the suture securing device; and
a suture clamping means within said elongate hollow body,
suture clamping means adapted to securely engage the free end of suture which has been passed through the loop in said suture securing device.

It will be understood that the delivery apparatus may be capable of releasably engaging a suture securing device, drawing a loop of suture through the passage in the securing device and clamping a suture.

The elongate hollow body of the delivery apparatus may include a projecting member at one end adapted to releasably engage with corresponding locating means on the suture securing device.

Preferably the projecting member includes a pair of arms adapted to engage corresponding locating recesses in the suture securing device. More preferably the arms include times resiliently biased towards engagement with the suture securing device.

The elongate hollow body may include a handle or the like at its other end. Preferably the suture recovery means and/or said suture clamping means are actuated in the region of said handle. More preferably the suture recovery means and/or suture clamping means are retractable along the length of the elongate body.

Accordingly the delivery apparatus may further include means for releasing said tines. In one embodiment the releasing means may include a ram, which acts upon cams located on the tines. When the ram contacts the cams, the tines are forced apart to release the device held thereby. Preferably the ram is actuated by a plunger, or other suitable means at the handle end of the delivery apparatus.

In this embodiment the apparatus may be provided in combination with integral suture recovery means and/or suture clamping means, or without said suture recovery means and/or suture clamping means.

The suture clamping means may include two cooperating members. The suture clamping means may include a delivery suture securing apparatus wherein said suture clamping means includes an abutment portion and a tongue member adapted to bear upon the abutment portion such that when a suture is passed through the loop, the tongue is pressed against the suture to hold it securely against the abutment portion.

The suture recovery means according to this aspect of the present invention may include any suitable arrangement for drawing a suture through the passage in the suture securing device. The suture recovery means may include a snare shaped to pass through the passage in the suture securing device and retractable through said passage such that when said snare is passed through said passage and a section of suture is snared, the snare may be retracted through said passage bringing with it a loop of said suture.

The snare may include a loop of filament.

The suture recovery means may then be capable of being disengaged from the loop or suture drawn through the suture securing device. Accordingly the snare may be attached to a member removably connectable to a retractable shaft, and is severable from said member when said shaft is retracted along said apparatus such that the snare is disengageable from said loop of suture recovered through the bore when the shaft is retracted along said apparatus.

In another embodiment the suture recovery means includes a securable thread attached to a disposable member attachable to a shaft, the thread being frangibly attached to the shaft. The suture recovery means may be automated to load the loop of suture and sever the thread in one action by actuating a lever or the like.

A method of securing a suture at a desired surgical site includes
providing a suture securing device for intracorporeally securing a suture including;
   a surgically implantable body having a tissue abutting side and a suture loop side; and
   a first suture passage extending through the body, said passage extending from an opening in the tissue abutting side to an opening in the suture loop side;
      said passage being shaped such that when a loop of suture is positioned within the passage and extending beyond the suture loop side and a free end of suture is passed through said loop, the loop clamps upon the free end when said loop is retracted back into said passage;
a delivery apparatus including
   an elongate hollow body at one end releasably engaging said suture securing device;
   a suture recovery means within said elongate hollow body adapted to draw a loop of suture through the first suture passage; and
   a suture clamping means within said elongate hollow body, adapted to securely engage suture which has been passed through the loop in said suture securing device; and
delivering said suture securing device to the desired surgical site; and
   a length of suture having a free end passing the suture recovery means through the first suture passage from the suture loop side to the tissue abutting side;
   snaring the suture to be secured with the suture recovery means;
   having a loop of suture through the passage;
   passing the free end of said suture around the device and through the loop;
   retracting the suture recovery means to release the loop;
   clamping the free end of the suture with the suture clamping means;
   retracting the clamping means to draw the suture taut about said suture securing device.

The method may further includes retracting the suture recovery means to disengage the suture loop; and
retracting the delivery apparatus to disengage said suture securing device.

The present invention will be described in more detail with reference to the accompanying drawings. It is to be understood that the drawings and the following description are illustrated only, and not intended to limit the generality of the present invention described above.

Figure 1 is an end elevation of a suture securing device showing particular aspects of the present invention, however, not the suture passage of the present invention.

Figure 2 is a side elevation of the suture securing device of Figure 1.

Figure 3 is a perspective view of a second embodiment of the suture securing device.

Figure 4 is a transparent perspective view of the suture securing device of Figure 3.

Figure 5 is a transverse section along line V-V through the device of Figure 4.

Figure 6 is a perspective view of a third embodiment of the suture securing device.

Figure 7 is a perspective view of a fourth embodiment of the suture securing device made in accordance with yet another aspect of the invention with two sutures in place.

Figure 8 is a side elevation of a delivery apparatus to be used in combination with the present invention.

Figure 9 is a plan of portion of the member of Figure 8 which holds the suture securing device.

Figure 10 is a side elevation of a suture clamping means according to this aspect of the present invention.

Figure 11 is a plan view of the delivery apparatus of Figure 10.

Figure 12 is a plan view of a suture recovery means according to this aspect of the present invention.

Figure 13 is a side elevation of the suture recovery means of Figure 12.

Figure 14 is a detailed view of the suture recovery means within the delivery apparatus.

Figures 15 to 19 are plan views of the delivery apparatus at various stages of operation.

Figure 20 is a plan view of a delivery apparatus shown holding a suture securing device.

Figure 21 is a plan view of the apparatus in Figure 20 at the point of release.

Figure 22 is a plan view of the apparatus with the ram retracted.

Figure 23 is a perspective view of a portion of the ram showing its end section.

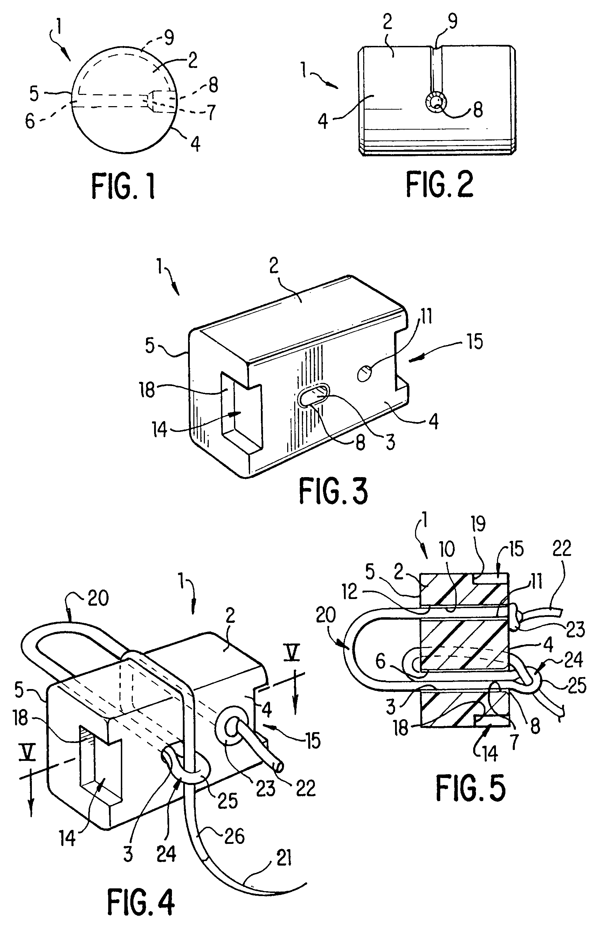

As best illustrated in Figures 1 and 2, suture securing device 1 includes a surgically implantable body 2 having a first suture passage extending therethrough. Device 1 may be any suitable shape such as a cylinder, sphere, cube, rectangular prism or the like but preferably the device is a rectangular prism. When the device is a rectangular prism preferably the external edges of the prism are radiussed in order to prevent abrasion of the suture when connected to the device and to prevent damage to surrounding body tissues once the device is implanted.

Body 2 has a tissue abutting side 5 which is suitable to bear against a body tissue when a suture is secured by device 1. Body 2 also has a suture loop side 4. Suture passage passes through body 2 and has openings 6 and 7 in the tissue abutting side 5 and suture loop side 4 respectively. At opening 7 in suture loop side 4 there is provided a tapered recess 8 shaped so that a loop of suture emerging from the passage and which has a portion of suture passed therethrough will clamp upon the portion of suture if the loop is retracted back into the passage.

A suture accommodating channel 9 is provided in the outside wall of body 2. Suture accommodating channel 9 passes between openings 6 and 7. Two suture accommodating channels (not shown) may be provided, one passing over the top of body 2 between opening 6 and 7 and another channel passing below body 2 between opening 6 and 7. Channel 9 is configured such that when a suture is located in the channel 9 and device 1 is held in a surgical clamp about the region of channel 9, the suture is still able to freely pass beneath the clamp.

In Figures 3, 4 and 5, the passage 3 according to the present invention is represented. In the embodiment illustrated there is also provided a second suture passage 10 which passes between tissue abutting portion 5 and suture loop portion 4 and has openings 11 and 12 adjacent portions 5 and 4. In Figure 3, second suture passage 10 is adapted to accommodate a single width of suture, i.e. the diameter of second suture passage 10 is approximately equal to the diameter of the suture. Opening 11 may be provided with a knot locating recess (not shown) adapted to locate a knot tied in the suture.

Body 2 is further provided with locating recesses 14 and 15 adapted to co-operate with corresponding projecting arms on a delivery apparatus of the type discussed below. Locating recesses 14 and 15 may be positioned at opposite ends of device 1. Recesses 14 and 15 may include walls 18 and 19 which are adapted to be contacted by the projecting arms on the delivery apparatus.

Device 1 can be used to secure a suture for a variety of surgical procedures. In the embodiment illustrated in Figures 3 to 5 a single device according to the invention can be used to form a secure loop such as for ligation or the like. In this embodiment a suture 20 is selected, preferably with surgical needle 21 attached. For example suture 20 may be any selected size of catgut, chromic or plain suture or synthetic, absorbable or non-absorbable, monofilament or braided suture or the like. Distal end 22 of suture 20 is first passed through the second suture passage 10 and a knot 23 is tied in distal end 20. Knot 23 may then be drawn towards a knot locating recess (not shown) and seated therein. The diameter of the second suture passage 10 will only accommodate a single portion of suture 20 and knot 23 cannot be drawn through passage 10. Suture 20 may then be used to snare or ligate tissue, tubes or vessels or suture tissue or prostheses. To secure the needle end of suture 20 a loop 24 of suture 20 is drawn through suture passage 3 from the tissue abutting side 5 of body 2 until the head 25 of loop 24 projects beyond opening 7. Loop 24 may be drawn through suture passage 3 by any suitable means as discussed below. The portion of suture 20 overlying body 2 between opening 6 and 7 may locate in channel 9. Device 1 may then be positioned at the desired surgical site.

In another embodiment of the invention, two devices in accordance with the invention may be used to secure a suture along an elongate wound. A first device is attached to the distal end of a suture. The first device need not include a second suture passage. A portion of the distal end of a suture is drawn through suture passage 5 until a loop head 25 projects beyond body 2. The distal end of the suture is then threaded through the head 25 of the loop. Suturing can then commence and the suture drawn through the body tissue until the tissue abutting portion 5 comes into contact with the body tissue. The distal end of the suture can not be drawn through the body tissue. Suturing can continue along the elongate wound away from the first device.

After the wound has been completely closed a second suture securing device may be attached about the proximal end of the suture i.e. by drawing a loop of suture through suture passage 3, passing the needle and adjacent suture through the head of the loop, drawing the suture between the needle and loop head taut and pushing the device along the suture towards the body tissue until the device comes into contact with and is secure against the body tissue. The suture is then severed and the needle and severed suture removed from the operating site.

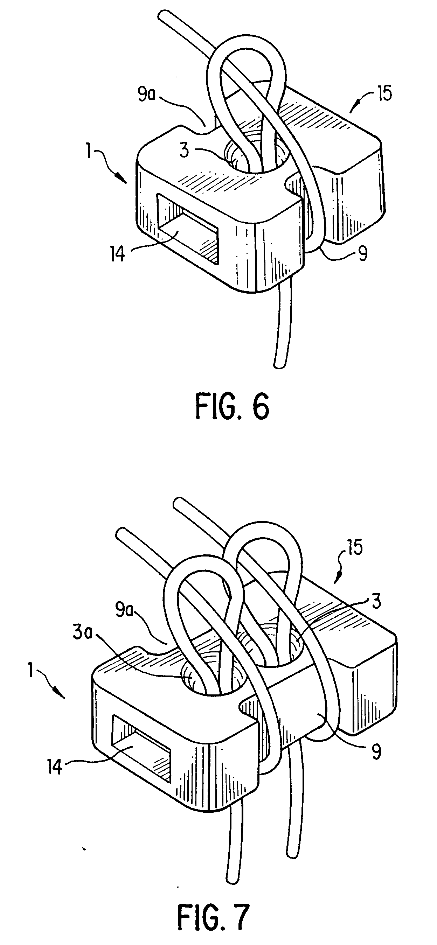

In Figure 6 device 1 has a single passage 3 therethrough. Device 1 has two channels 9 and 9a in its outside wall. Channels 9 and 9a are substantially parallel to passage 3.

Device 1 has locating recesses 14 and 15 adapted to locate corresponding projecting arms on an apparatus for positioning the device.

In Figure 7 device 1 has a pair of passages 3 and 3a adapted to secure two sutures or two ends of one suture. Device 1 has channels 9 and 9a in its outside wall which accommodate portions of both sutures being secured.

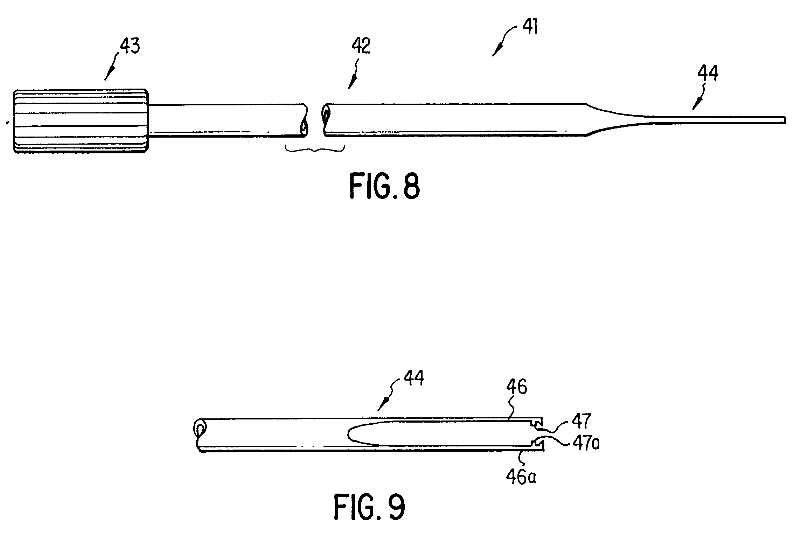

As illustrated in Figure 8 and 9 delivery apparatus 41 comprises an elongate hollow body 42 having at one end a handle 43. Handle 43 is configured to allow a surgeon to easily manipulate the delivery apparatus 41 during surgery. At the other end of elongate member 42 there is provided projecting member 44 adapted to hold a suture securing device 1. Projecting member 44 includes a pair of tines 46, 46a having lugs 47 and 47a adapted to locate in corresponding recesses in device 1. Elongate hollow body 42 is suitably configured for use in endoscopic surgery i.e. at least a portion of elongate body 42 is capable of being passed down a portal to the desired operating site. Tines 46 and 46a are resiliently biased towards a position where they firmly engage a suture securing device 1 positioned therebetween.

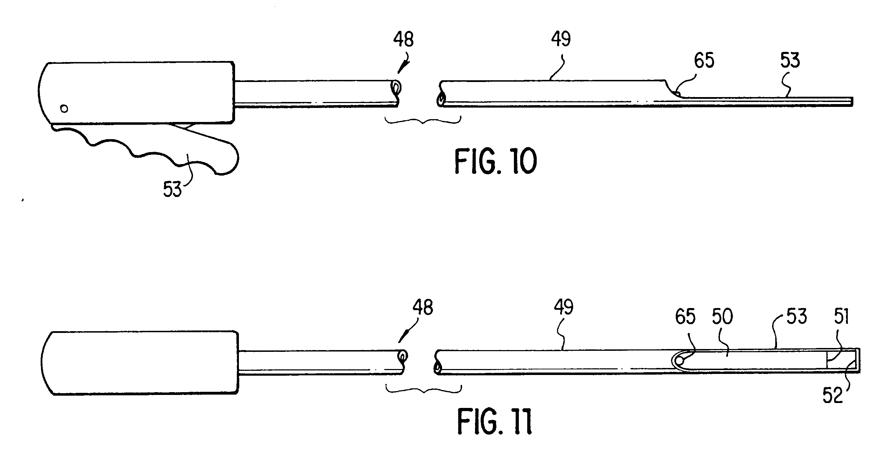

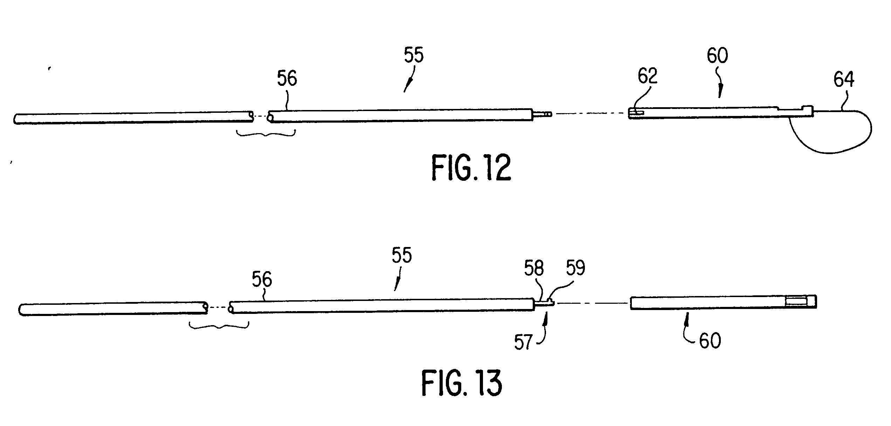

As illustrated in Figures 10 and 11, the delivery apparatus 41 further includes a suture clamping means 48. Clamping means 48 consists of two cooperating members 49 and 50 which co-act to grasp and firmly hold a suture which has been passed between clamp face 51 on member 50 and claimp facd 52 on member 49. Cooperating member 49 is an elongate tubular body with a partial cut-away end 53. Cooperating member 50 is accommodated in an axial bore through member 49. Clamp face 51 may be a tongue on member 50 adapted to bear upon clamp face 52 of member 49. Suture clamping means 48 further includes an actuating mechanism 53 and capable of moving clamp faces 51 and 52 relative to each other to clamp a suture passed between the faces. Actuating mechanism 53 includes a lever, which, when depressed by the surgeon moves cooperating members 49 and 50 relative to each other and brings clamp faces 51 and 52 together. Suture clamping means 48 is positioned within elongate hollow body 42. The suture clamping means further includes incising edge 65 as discussed below.

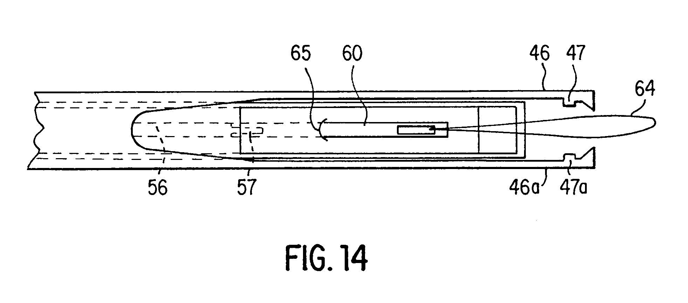

As illustrated in Figures 12, 13 and 14, delivery apparatus 41 further includes a suture recovery means 55 adapted to draw a loop of suture through the suture passage 3 in suture securing device 5. Suture snare means 55 consists of a shaft 56 positioned within suture clamping means 48. Shaft 56 is connected to a replaceable head 60 via coupling means 57. Coupling means 57 consists of a finger 58 which projects from the end of shaft 56 and lug 59. Replaceable head 60 includes a recess 62 adapted to receive coupling means 57. A frangible loop 64 is attached to head 60 such that when the suture snare is retracted into the bore, frangible loop 64 is severed. Loop 64 may be severed by a scissor action between the head portion 60 and an incising edge 65 at bore opening of suture clamping means 48. The suture recovery means 55 is configured such that shaft 56 cannot be fully retracted from apparatus during use and stops such that the tip of head portion is retained within the bore such that the gas seal is not broken. The snare loop 64 may be formed of any suitable flexible material. Snare loop 64 may itself be made of suture material.

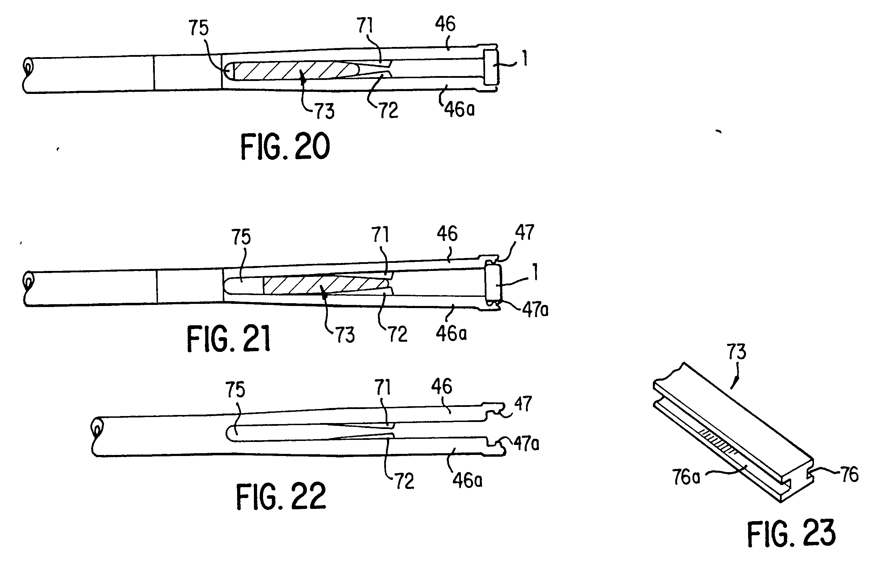

In the embodiment shown in Figures 20 to 23 tines 46 and 46a carry cams 71 and 72 which interact with ram 73 at the end of a shaft 75. When a plunger or other actuating means at the handle end (not shown) of the delivery apparatus is actuated, force is transmitted along shaft 75 to drive ram 73 onto rams 71 and 72 as shown in Figure 21. Tines 46 and 46a and hence lugs 47 and 47a are forced apart to disengage from corresponding recesses in device 1. A section of ram 73 is illustrated in Figure 23. Ram 73 has cam grooves 76 and 76a such that ram 73 positively engages cams 71 and 72 when actuated.

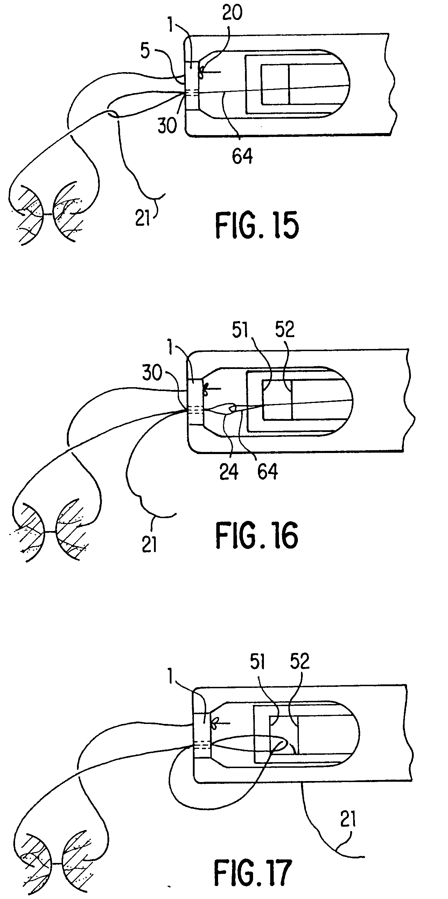

Figures 16 to 19 illustrate the positioning a suture securing device 1 at a desired surgical site. Where a single suture securing device 1 is shown with one end of a suture 20 securely affixed thereto and emerging from said device adjacent a tissue abutting face 27. The free end of the suture terminates in a suturing needle 21. In a preferred embodiment, suture securing device 1 is provided with suture 20 attached and with frangible loop 64 pre-positioned through suture passage 3.

Once suturing has been completed needle 21 is passed through the frangible loop 64 as shown in Figure 15. Suture 20 is then drawn through passage 3 as shown in Figure 16 by retracting the suture recovery means 55 until a loop of suture 24 emerges on the apparatus side of device 1. Suture loop 24 is drawn until it is adjacent clamp faces 51 and 52. Further retraction of suture recovery means 55 severs snare loop 64 against incising edge 65.

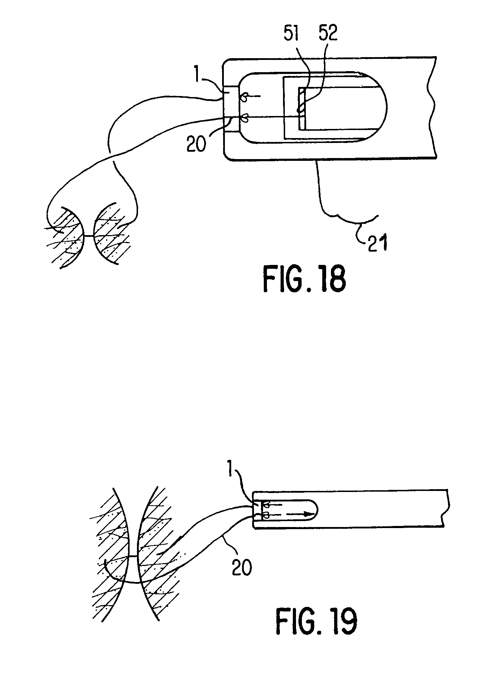

Needle 21 is then passed through suture loop 24 and between clamp faces 51 and 52 as shown in Figure 17. By depressing actuating lever 53, clamp faces 51 and 52 clamp the suture as shown in Figure 18. Suture clamping means 48 may then be retracted relative to device 1 to tension suture 20, as shown in Figure 18. Finally, in order to position suture securing device 1 at the desired site, delivery apparatus 41 is pushed towards the desired site whilst suture clamping means 48 draws slack suture through device 1 towards the tissue against which it is to hold the suture 20. Once at the desired site, the elongate body 2 can be retracted and the secure hold of device 1 enables tines 46, 46a to release the device when retracted. Excess suture material can then be severed.

If the suture securing device 1 is to secure only one end of suture 20, the device need not be provided with the pre-attached suture as previously described. The suture securing device 1 need only be provided with one bore.

The delivery apparatus 41 may further be utilized to position a suture securing device 1 for ligation of a vessel, organ, duct, tissue or the like. A suture securing device 1 may be provided pre-tied with a suture and loaded into apparatus 1 in the form shown in Figure 18. The suture 20 need not be provided with a needle. The loop of suture emerging from the tissue abutting side of the device 1 may simply be slipped around the tissue, vessel, organ, duct or the like in a lasso fashion, rather than being passed through the tissue as shown in Figure 18. The device 1 may be positioned to ligate the desired site in a manner identical to that described above, i.e. by clamping the free end of the suture between clamp faces 51 and 52.

Preferably, suture recovery means 55 and suture clamping means 48 are located within the elongate hollow body 42.

In the embodiment as shown in Figures 19, 21, 22 and 23, the apparatus may be provided without integral suture snare means and/or suture clamping means. If provided without such means, the suture may be threaded about the device using suitable conventional surgical instruments. If provided with such means, preferably the holding member, suture recovery means and suture clamping means are supplied as an integral unit capable of insertion down a single port for endoscopic surgery.

It will be readily apparent that a plurality of suture securing devices may be used during a surgical procedure. As different strengths and thicknesses of suture may be used by a surgeon it will be appreciated that a range of suture securing devices may be provided with different sized suture passages appropriate for corresponding suture sizes. The suture securing device may be any suitable size according to the desired application. The suture securing device may be made from any suitable material which is acceptable for implantation into the body of a patient. The device may be made from a non-absorbable polymeric material, for example high density polyethylene or a plastic sold under the trade mark DELRIN. The device may be made from a bio-absorbable material which is known in the art such as polyglycolic acid.

The device may be manufactured by any suitable method known in the art. The device may be made by injection moulding. The device may be made under aseptic conditions and packaged in a sterile form immediately after manufacture or may be manufactured under non-sterile conditions and subsequently sterilized by any suitable means.

The delivery apparatus may be made from any suitable materials by any suitable method. Preferably, the elongate hollow body 42, suture clamping means 48 and shaft 56 are made from surgical steel which can be sterilized and re-used a number of times. Head 60 may be disposable and made from a suitable sterilizable polymeric material. The snare loop may be any suitable flexible wire, filament or the like. Preferably when a disposable head is used and the loading loop is to be separable, the loading loop is made from a narrow gauge suture material or the like.

It is to be understood that various modifications, additions and/or alterations may be made to the parts previously described without departing from the scope of the present invention as defined in the appended claims.

## Claims

1. A suture securing device (1) for intracorporeally securing a suture including:
a surgically implantable body (2) having a tissue abutting side (5) and a suture loop side (4); at least a first suture passage (3) extending from an opening (6) in the tissue abutting side (5) to an opening (7) in the suture loop side (4) characterized by the fact that in transverse cross-section, said passage (3) is provided with a height generally equal to a diameter of said suture and a width generally twice a diameter of said suture such that when a loop of suture (24) is positioned within said first passage (3) end extending beyond the suture loop side (4) and a free end of suture is passed through said loop (24), the loop clamps upon the free end when said loop (24) is retracted back into said passage.

2. A suture securing device (1) according to claim 1 wherein the surgically implantable body (2) includes an outer peripheral surface, and wherein the device (1) further includes a suture accommodating channel (9) in the outer peripheral surface extending substantially parallel to said passage (3), said channel (9) being disposed about a portion of said outer peripheral surface, such that a suture accomodated in the channel (9) is not gripped when said body (2) is gripped about a portion of said outer peripheral surface.

3. A suture securing device (1) according to claim 1 further including a second suture passage (10) extending through said body (2), said second suture passage (10) substantially parallel to said first suture passage (3), wherein transverse cross-section of said second passage (10) is provided with a diameter generally equal to the diameter of a single suture so that a suture thread passing therethrough and being knotted is prevented from return passage therethrough.

4. A suture securing device (1) according to claim 1 further including locating means for delivering said device in use to a surgical side, said locating means comprising a pair of recesses (14, 15) located at opposite ends of the surgically implanted body and shaped to receive a cooperating portion of an apparatus (41) upon engagement of said apparatus (41) and a surface portion of the suture loop side disposed to receive axial force delivered by said apparatus (41) to bring the tissue abutting side into abutment with the tissue.

5. An apparatus comprising a suture securing device according to claim 1 and additionally a suture recovery means (55) including a snare (64) shaped to pass through the passage (3) in the suture securing device and retractable through said passage (3) such that when said snare is passed through said passage (3) and a section of suture is snared, the snare may be retracted through said passage (3) bringing with it a loop of said suture.

6. An apparatus comprising a suture securing device (1) according to claim 1 and additionally an elongate hollow body (42) adapted at one end to releasably engage said suture securing device (1);
a suture recovery means (55) within said elongate hollow body (42) adapted to draw a loop of suture through the first suture passage (3) of the suture securing device (1); and
a suture clamping means (48) within said elongate hollow body (42), adapted to securely engage suture which has been passed through the loop in said suture securing device (1).

7. An apparatus according to claim 6 wherein the elongate hollow body (42) includes a projecting member having a pair of arms (46, 46a) adapted to engage corresponding locating recesses in the suture securing device (1) and resiliently biased towards engagement with the suture securing device (1).

8. An apparatus according to claim 7 wherein the suture recovery means (55) and/or suture clamping means (48) are retractable along the length of the elongate body (42).

9. An apparatus according to claim 6 wherein said suture clamping means (48) includes an abutment portion (52) and a tongue member (51) adapted to bear upon the abutment portion (52) such that when a suture is passed through the loop, the tongue (51) is pressed against the suture to hold it securely against the abutment portion (52).

10. An apparatus according to claim 6 wherein the suture recovery means (55) includes a snare (64) consisting of a loop of filament shaped to pass through the passage (3) in the suture securing device (1) and retractable through said passage (3) such that when said snare (64) is passed through said passage (3) and a section of suture is snared, the snare (64) may be retracted through said passage (3) bringing with it a loop (24) of said suture and wherein the snare (64) is attached to a member (60) removably connectable to a retractable shaft, and is severable from said member (60) when said shaft is retracted along said apparatus (41) such that the snare (64) is disengageable from said loop of suture (24) recovered through the bore when the shaft is retracted along said apparatus (41).

## Patentansprüche

1. Vorrichtung (1) zur Befestigung von Nahtmaterial zum Befestigen von Nahtmaterial innerhalb eines Körpers, mit einem chirurgisch implantierbaren Körper (2) mit einer Gewebeanlageseite (5) und einer Nahtmaterialschlaufe-Seite (4), wobei sich mindestens ein erster Durchgang (3) für Nahtmaterial von einer Öffnung (6) auf der Gewebeanlageseite (5) zu einer Öffnung (7) auf der Nahtmaterialschlaufe-Seite (4) erstreckt, **dadurch gekennzeichnet, dass** der Durchgang (3) im Querschnitt mit einer Höhe, die im wesentlichen dem Durchmesser des Nahtmaterials entspricht, und mit einer Breite versehen ist, die im wesentlichen das Doppelte des Durchmessers des Nahtmaterials ist, so dass dann, wenn eine Nahtmaterialschlaufe (24) innerhalb des ersten Durchgangs (3) positioniert wird und sich über die Nahtmaterialschlaufe-Seite (4) hinaus erstreckt, und das freie Ende des Nahtmaterials durch diese Schlaufe (24) geführt wird, die Schlaufe das freie Ende festklemmt, wenn diese Schlaufe wieder in den Durchgang zurückgezogen wird.

2. Vorrichtung (1) zur Befestigung von Nahtmaterial nach Anspruch 1, bei der der chirurgisch implantierbare Körper (2) eine Außenumfangsfläche aufweist, und die ferner einen Nahtmaterial-Aufnahmekanal (9) an der Außenumfangsfläche aufweist, der sich im wesentlichen parallel zum Durchgang (3) erstreckt und um einen Abschnitt der Außenumfangsfläche herum so angeordnet ist, dass in diesem Kanal (9) aufgenommenes Nahtmaterial nicht erfasst wird, wenn der Körper (2) an einem Abschnitt dieser Außenumfangsfläche erfasst wird.

3. Vorrichtung (1) zur Befestigung von Nahtmaterial nach Anspruch 1, ferner mit einem zweiten Durchgang (10) für Nahtmaterial, der sich durch den Körper (2) im wesentlichen parallel zum ersten Durchgang (3) für Nahtmaterial erstreckt, wobei der Querschnitt diesen zweiten Durchgangs (10) mit einem Durchmesser versehen ist, der im wesentlichen dem Durchmesser eines einzelnen Nahtmaterials entspricht, so dass ein durch ihn hindurchlaufender und verknoteter Nahtmaterialknopf daran gehindert ist, durch den Durchgang zurückzulaufen.

4. Vorrichtung (1) zur Befestigung von Nahtmaterial nach Anspruch 1, ferner mit einer Positioniereinrichtung zum Zuführen der Vorrichtung zu einem Operationsort, wobei diese Positioniereinrichtung ein Paar Aussparungen (14, 15) aufweist, die an entgegengesetzten Enden des chirurgisch implantierten Körpers liegen und so geformt sind, dass sie einen zusammenwirkenden Abschnitt eines Geräts (41) aufnehmen, wenn Eingriff mit diesem Gerät (41) besteht, und wobei ein Oberflächenabschnitt der Nahtmaterialschlaufen-Seite so angeordnet ist, dass er eine vom Gerät (41) aufgebrachte axiale Kraft aufnimmt, um die Gewebeanlageseite zur Anlage an Gewebe zu bringen.

5. Gerät mit einer Vorrichtung zur Befestigung von Nahtmaterial nach Anspruch 1 und zusätzlich mit einer Nahtmaterial-Wiedererlangungseinrichtung (55) mit einer Fangeinrichtung (64), die so ausgebildet ist, dass sie durch den Durchgang (3) in der Vorrichtung zur Befestigung von Nahtmaterial hindurchgeht und durch diesen Durchgang (3) zurückziehbar ist, so dass dann, wenn die Fangeinrichtung durch diesen Durchgang (3) hindurchgeführt wird und ein Abschnitt des Nahtmaterials eingefangen wird, die Fangeinrichtung durch den Durchgang (3) zurückgezogen werden kann, wobei sie eine Schlaufe des Nahtmaterials mit sich führt.

6. Gerät mit einer Vorrichtung (1) zur Befestigung von Nahtmaterial nach Anspruch 1 und mit zusätzlich einem langgestreckten, hohlen Körper (42), der an einem Ende so ausgebildet ist, dass er lösbar mit der Vorrichtung (1) zur Befestigung von Nahtmaterial in Eingriff tritt, mit
- einer Nahtmaterial-Wiedererlangungseinrichtung (55) innerhalb des langgestreckten, hohlen Körpers (42), die so ausgebildet ist, dass sie eine Nahtmaterialschlaufe durch den ersten Durchgang (3) für Nahtmaterial in der Vorrichtung (1) zur Befestigung von Nahtmaterial hindurchzieht; und
- einer Nahtmaterial-Klemmeinrichtung (48) innerhalb des langgestreckten, hohlen Körpers (42), die so ausgebildet ist, dass sie fest mit Nahtmaterial in Eingriff tritt, das durch die Schlaufe in der Vorrichtung (1) zur Befestigung von Nahtmaterial geführt wurde.

7. Gerät nach Anspruch 6, bei dem der langgestreckte, hohle Körper (42) ein vorstehendes Element mit einem Paar Arme (46, 46a) aufweist, die so ausgebildet sind, dass sie in entsprechende Positionieraussparungen in der Vorrichtung (1) zur Befestigung von Nahtmaterial eingreifen, und die elastisch zum Eingriff in diese Vorrichtung (1) zur Befestigung von Nahtmaterial vorgespannt sind.

8. Gerät nach Anspruch 7, bei dem die Nahtmaterial-Wiedererlangungseinrichtung (55) und/oder die Nahtmaterial-Klemmeinrichtung (48) entlang der Länge des langgestreckten Körpers (42) zurückziehbar sind.

9. Gerät nach Anspruch 6, bei dem die Nahtmaterial-Klemmeinrichtung (48) einen Anlageabschnitt (52) und ein Zungenelement (51) aufweist, das so ausgebildet ist, dass es auf dem Anlageabschnitt (52) ruht, so dass dann, wenn Nahtmaterial durch die Schlaufe geführt ist, die Zunge (51) gegen das Nahtmaterial drückt, um es fest am Anlageabschnitt (52) zu halten.

10. Gerät nach Anspruch 6, bei dem die Nahtmaterial-Wiedererlangungseinrichtung (55) eine Fangeinrichtung (64) aus einer Faserschlaufe aufweist, die so geformt ist, dass sie durch den Durchgang (3) in der Vorrichtung (1) zur Befestigung von Nahtmaterial hindurchgeht und durch diesen Durchgang (3) zurückziehbar ist, so dass dann, wenn diese Fangeinrichtung (64) durch den Durchgang (3) hindurchgeführt ist und ein Nahtmaterialabschnitt eingefangen ist, die Fangeinrichtung (64) durch den Durchgang (3) zurückgezogen werden kann, wobei sie eine Schlaufe (24) des Nahtmaterials mit sich führt, und wobei die Fangeinrichtung (64) an einem Element (60) befestigt ist, das lösbar mit einem zurückziehbaren Schaft verbunden ist, und sie von diesem Element (60) abtrennbar ist, wenn der Schaft entlang dem Gerät (41) zurückgezogen wird, so dass die Fangeinrichtung (64) von der Nahtmaterialschlaufe (24) lösbar ist, die durch die Bohrung zurückgezogen wurde, wenn der Schaft entlang dem Gerät (41) zurückgezogen wird.

## Revendications

1. Dispositif d'immobilisation d'un fil de suture (1) destiné à immobiliser à l'intérieur du corps un fil de suture comprenant :
un corps implantable chirurgicalement (2) avant un côté attenant au tissu (5) et un côté fil de suture en boucle (4); au moins un premier passage de fil de suture (3) s'étendant depuis une ouverture (6) dans le côté attenant au tissu (5) jusqu'à une ouverture (7) dans le côté fil de suture en boucle (4), caractérisé par le fait qu'en coupe transversale, ledit passage (3) présente une hauteur globalement égale au diamètre dudit fil de suture et une largeur globalement égale à deux fois le diamètre dudit fil de suture de telle façon que lorsqu'une bouche de fil de suture (24) est positionnée à l'intérieur dudit passage (3) s'étendant au-delà du côté fil de suture en boucle (4) et qu'une extrémité libre du fil de suture traverse ladite boucle (24), la boucle se resserre sur l'extrémité libre lorsque ladite boucle (24) est retirée dudit passage.

2. Dispositif d'immobilisation de fil de suture (1) selon la revendication 1, dans lequel le corps chirurgicalement implantable (2) comprend une surface périphérique extérieure, et dans lequel le dispositif (1) comprend en outre une rainure logeant le fil de suture (9) dans la surface périphérique extérieure s'étendant pratiquement parallèlement audit passage (3), ladite rainure (9) étant disposée autour d'une partie de ladite surface périphérique extérieure de telle façon qu'un fil de suture logé dans la rainure (9) n'est pas saisi lorsque ledit corps (2) est accroché autour d'une partie de ladite surface périphérique extérieure.

3. Dispositif d'immobilisation de fil de suture (1) selon la revendication 1, comprenant en outre un second passage de fil de suture (10) s'étendant à travers ledit corps (2), ledit second passage de fil de suture (10) étant pratiquement parallèle audit premier passage de fil de suture (3), dans lequel la coupe transversale dudit second passage (10) présente un diamètre globalement égal au diamètre d'un fil de suture unique de telle façon qu'un fil de suture qui le traverse et qui est noué est empêché de le retraverser.

4. Dispositif d'immobilisation de fil de suture (1) selon la revendication 1, comprenant en outre un moyen de repérage pour amener ledit dispositif devant être utilisé du côté chirurgie, ledit moyen de repérage étant constitué d'une paire d'encoches (14, 15) situées aux extrémités opposées du corps chirurgicalement implanté et formées pour recevoir la partie coopérante d'un appareil (41) lors de l'introduction dudit appareil (41), et une partie formant surface du côté bouche de fil de suture disposée pour recevoir la force axiale fournie par ledit appareil (41) pour amener le côté attenant au tissu à venir en butée contre le tissu.

5. Appareil comprenant un dispositif d'immobilisation de fil de suture selon la revendication 1, et de plus un moyen de récupération du fil de suture (55) comportant un noeud coulant (64) formé pour traverser le passage (3) du dispositif d'immobilisation de fil de suture et qui peut se retirer dudit passage (3) de telle façon que lorsque ledit noeud coulant traverse ledit passage (3) et qu'un troncon de fil de suture est formé en noeud coulant, le noeud coulant peut être retiré dudit passage (3) en amenant avec lui une boucle dudit fil de suture.

6. Appareil comprenant un dispositif d'immobilisation de fil de suture (1) selon la revendication 1 et de plus un corps creux allongé (42) adapté à l'une des extrémités pour s'engager, de façon à pouvoir être démonté, avec ledit dispositif d'immobilisation de fil de suture (1);
un moyen de récupération de fil de suture (55) à l'intérieur dudit corps creux allongé (42) adapté pour tirer une boucle de fil de suture à travers le premier passage de fil de suture (3) du dispositif d'immobilisation de fil de suture (1); et
un moyen de serrage de fil de suture (48) à l'intérieur dudit corps creux allongé (42), adapté pour engager fermement le fil de suture qui a traversé la boucle dans ledit dispositif d'immobilisation de fil de suture (1).

7. Appareil selon la revendication 6 dans lequel le corps creux allongé (42) comprend un élément en saillie comportant une paire de bras (46, 46a) adaptés pour s'engager dans les encoches correspondantes de logement du dispositif d'immobilisation de fil de suture (1), et qui est rappelé élastiquement pour engager le dispositif d'immobilisation de fil de suture (1).

8. Appareil selon la revendication 7, dans lequel le moyen de récupération de fil de suture (55) et/ou le moyen de serrage de fil de suture (48) peuvent être retirés le long de l'élément de corps allongé (42).

9. Appareil selon la revendication 6, dans lequel ledit moyen de serrage de fil de suture (48) comprend une partie formant butée (52) et un élément languette (51) adapté pour s'appuyer sur la partie formant butée (52), de telle façon que lorsqu'un fil de suture traverse la boucle, la languette (51) est poussée contre le fil de suture pour le maintenir fermement contre la partie formant butée (52).

10. Appareil selon la revendication 6, dans lequel le moyen de récupération de fil de suture (55) comprend un noeud coulant (64) constitué d'une boucle de filament formée pour traverser le passage (3) du dispositif d'immobilisation de fil de suture et qui petit être retiré du passage (3) de telle façon que lorsque ledit noeud coulant (64) a traversé ledit passage (3) et qu'un tronçon de fil de suture est formé en noeud coulant, le noeud coulant (64) peut être retiré par ledit passage (3) en amenant avec lui une boucle (24) dudit fil de suture et dans lequel le noeud coulant (64) est attaché à un élément (60) qui peut être relié, de façon à pouvoir être enlevé, à un arbre rétractable, et il peut être séparé dudit élément (60) lorsque ledit arbre est rétracté le long dudit appareil (41) de telle façon que le noeud coulant (64) peut être désengagé de la boucle de fil de suture (24) récupérée par le trou lorsque l'arbre est rétracté le long dudit appareil (41).
